Europäisches Patentamt

European Patent Office

Office européen des brevets

(11) Publication number: **0 372 928**
**A2**

(12) **EUROPEAN PATENT APPLICATION**

(21) Application number: **89312713.4**

(22) Date of filing: **06.12.89**

(51) Int. Cl.⁵: **C12N 15/62, C12N 15/31,**
**C07K 15/00, A61K 39/116**

(30) Priority: **07.12.88 GB 8828523**
**17.06.89 GB 8913991**

(43) Date of publication of application:
**13.06.90 Bulletin 90/24**

(84) Designated Contracting States:
**AT BE CH DE ES FR GB GR IT LI LU NL SE**

(71) Applicant: **UNIVERSITY OF LEICESTER**
**University Road**
**Leicester, LE1 7RH(GB)**

(72) Inventor: **Hirst, Timothy Raymond**
**Adrian Building University Road**
**Leicester LE1 7RH(GB)**
Inventor: **Aitken, Rober**
**Adrian Building University Road**
**Leicester LE1 7RH(GB)**

(74) Representative: **Woods, Geoffrey Corlett et al**
**J.A. KEMP & CO. 14 South Square Gray's Inn**
**London WC1R 5EU(GB)**

(54) **Heat-labile toxin B subunit fusion proteins.**

(57) A fusion protein comprises subunit B of E. coli heat-labile toxin (LTB) having an antigen or epitope from a pathogen responsible for a human or veterinary disease fused to the carboxy-terminus of LTB. Such a fusion protein is obtained by culturing a host, such as a strain of E. coli, which has been transformed by a vector capable of expressing the fusion protein in that host. The fusion protein can be used as a vaccine.

EP 0 372 928 A2

# HEAT-LABILE TOXIN B SUBUNIT FUSION PROTEINS

The present invention relates to heat-labile toxin B subunit fusion proteins, to their preparation and to their use.

Toxigenic strains of Escherichia coli (ETEC) are among the most important causes of infant morbidity and mortality in the developing world. Any vaccine able to reduce the estimated 650 million cases (including about 800,000 deaths) per annum will clearly make a significant contribution to global health care. The development of such vaccines has to address the requirement to immunise against ETEC of different serotypes that produce a variety of adhesive antigens and one or both of two highly potent enterotoxins. It will also be necessary to evaluate the route and ease of vaccine administration, as well as vaccine storage, stability, and cost.

We have successfully generated a series of fusion proteins in which different portions of the gene for heat-stable enterotoxin (ST) are fused to the 3'-end of the gene encoding heat-labile enterotoxin B subunit (LTB). These LTB-ST recombinant hybrids possess all of the relevant properties for making them effective mucosal immunogens. We have also expressed another hybrid protein in which an epitope is fused to the carboxy-terminal of LTB. Fusion proteins in which an antigen or epitope is fused to the carboxy-end of LTB therefore represent a new way of effectively presenting the antigen or epitope to the immune system.

Accordingly, the present invention provides a fusion protein which comprises subunit B of E. coli heat-labile toxin (LTB) having an antigen or epitope from a pathogen responsible for a human or veterinary disease fused to the carboxy-terminus of LTB.

The fusion proteins fold correctly and assemble into stable pentamer complexes. Natural LTB assembles to holotoxin oligomers with adenylate-cyclase-activating subunit A in a molar ratio B:A of 5:1. The fusion proteins have a high affinity for binding to GM1-ganglioside, strong immunoreactivity toward neutralising anti-LT antibody and neutralising antibody to the antigen or epitope fused to the LTB and the capacity to induce antibody responses to LTB and the fused antigen or epitope.

The fusion proteins employ LTB as a carrier for an antigen or epitope. Sufficient structural information must be retained in the LTB gene to favour the formation of a chimaeric protein that binds to GM1-ganglioside and displays all LTB epitopes. Typically, therefore, the amino acid sequence of LTB up to amino acid 102 (Glu) is retained. The LTB may be human LTB (hLTB) or porcine LTB (pLTB), for example.

A linker sequence can be provided between the carboxy-terminus of LTB and the antigen or epitope fused to the LTB. The sequence may have up to 10 amino acid residues, for example 2 or 3 residues. A suitable linker sequence therefore is -Lys-Leu- or -Lys-Leu-Gly-.

The sequence at the joint between the LTB and the fused antigen or epitope preferably comprises -Lys-Leu-Gly-Pro-Gln-. The presence of this joint sequence is particularly beneficial in ensuring that the LTB portion of the fusion protein and the fused antigen or epitope both fold correctly and consequently are properly presented to the immune system of a human or animal to which they may be administered. Further, the joint sequence is also particularly beneficial in enabling the fusion protein to self-assemble into stable pentamers.

The joint sequence is provided where the fused antigen or epitope is joined to LTB. The joint sequence may comprise one or more of the carboxy-terminal amino acid residues of LTB and/or one or more of the amino-terminal amino acid residues of the fused antigen or epitope. Typically, the joint sequence follows immediately after the LTB amino acid sequence. The final two or three amino-terminal residues of the fused antigen or epitope may form part of the joint sequence.

Any amino acid sequence having biological activity may be fused to the carboxy-terminus of the LTB. Any antigen or epitope from a pathogen responsible for a human or veterinary disease may be presented by the fusion protein. The antigen or epitope may be one which is capable of effecting an immune response, e.g. raising neutralising or non-neutralising antibodies or cellular immunity. A predicted antigenic determinant may be employed. The epitope may be up to 60, for example up to 20 amino acid residues in length.

A foreign amino acid sequence is therefore fused to LTB. This may therefore comprise an antigenic determinant capable of raising neutralising antibody to a pathogenic organism. The epitope may be derived from a virus, bacterium, fungus, yeast or parasite. More especially, the epitope may be derived from a type of human immunodeficiency virus (HIV) such as HIV-1 or HIV-2, hepatitis A or B virus, human rhinovirus such as type 2 or type 14, herpes simplex virus, poliovirus type 2 or 3, foot-and-mouth disease virus, influenza virus, coxsackie virus, the cell surface antigen CD4, Chlamydia trachomatis, RSV, HPV e.g. HPV 16, and the heat-stable enterotoxin of E. coli (ST).

LTB may therefore be fused to an immunogenic portion of ST. The LTB can be fused to the core and toxin regions of ST or to the toxin region only. For example, LTB can be linked to a portion of ST beginning at ST amino acid number 19(Pro) or at ST amino acid number 49 (Gly). Both the long and short hybrid proteins which result include the entire nineteen carboxy-terminal amino acid residues of ST which contain the six Cys residues necessary for native ST configuration.

The fusion proteins are prepared by recombinant DNA methodology. More particularly, a fusion protein is prepared by a process which comprises culturing a host, which has been transformed with a vector capable of expressing the fusion protein in that host, under such conditions that the fusion protein is expressed. The fusion protein can then be isolated, typically in biologically pure form.

The preparation of the fusion protein therefore depends upon the provision of a DNA sequence encoding the fusion protein. The DNA sequence may be provided at its 5′-end with a sequence encoding a leader for the fusion protein so that the fusion protein is exported from the cytoplasm of the host cell in which it is expressed. Any appropriate leader sequence may be employed. Typically, however, DNA encoding the natural LTB leader sequence is provided immediately upstream of the DNA encoding the mature LTB sequence. sequence.

Thus a DNA sequence encoding the desired fusion protein is provided. An expression vector is prepared which incorporates the DNA sequence and which is capable of expressing the fusion protein when provided in a suitable host. Appropriate transcriptional and translational control elements are provided for the DNA sequence, in particular a promoter for the DNA sequence and a transcriptional termination site. The DNA sequence is located between translation start and stop signals in the vector. The DNA sequence is provided in the correct frame such as to enable expression of the fusion protein to occur in a host compatible with the vector.

The expression vector is used to transform a host. Cells harbouring the expression vector are grown so as to enable expression of the fusion protein to occur. The fusion protein may self-assemble into oligomers. Any appropriate host-vector system may be employed. The host may be a procaryotic or eucaryotic host. The vector may be plasmid. In that event, a bacterial or yeast host may be used for example a Gram negative bacillus such as E. coli or a Vibrio species, or S. cerevisiae. Alternatively, the vector may be a viral vector. This may be used to transfect cells of a mammalian cell line, such as Chinese Hamster Ovary (CHO) cells, in order to cause expression.

A fusion protein expression vector may be prepared by:
(a) providing a LTB expression vector; and
(b) ligating a gene encoding a desired antigen or epitope to the 3′-end of the LTB gene.

A vector capable of expressing LTB can be obtained by cloning the LTB gene (Dallas, Infect. Immun. 40, 647-652, 1983) into a vector under the control of appropriate transcriptional and translational regulatory elements. If the fusion protein has a linker sequence between the LTB and the antigen or epitope, oligonucleotides corresponding to the linker sequence can be synthesised and fitted to the 3′-end of the LTB gene or to the 5′-end of the gene encoding the antigen or epitope.

A preferred vector for use in obtaining a fusion protein expression vector is a vector which encodes LTB having a carboxy-terminal extension. The coding sequence for the extension is selected such that it has a restriction site. A gene encoding an antigen or epitope it is desired to fuse to LTB can be inserted at this site. The coding sequence up to the restriction site is also selected such that it encodes the correct amino acid residues for all or part of a desired linker sequence.

The invention therefore provides an expression vector which encodes LTB having a carboxy-terminal extension wherein the coding sequence for the extension has a restriction site therein.

The invention further provides a process for the preparation of a fusion protein comprising LTB and an antigen or epitope from a pathogen responsible for a human or veterinary disease fused to the carboxy-terminus of LTB, which process comprises (i) inserting a DNA sequence encoding the antigen or epitope into such a vector at the said restriction site; and (ii) culturing a host harbouring the resultant vector under such conditions that the fusion protein is expressed.

The restriction site can be provided in any reading frame. More than one site can be present. The carboxy-terminal extension can be of any length, for example 5 to 10 and especially 8 amino acid residues. Preferred extensions, their coding sequences and the location of a restriction site for NaeI (_) are:
Lys Leu Gly Pro Gln Ala Gly Asp ""
AAG CTG GGT CCG CAG GCC GGC GAC TAG
Lys Leu Gly Pro Gln Gly Arg His ""
AAG CTG GGT CCG CAG GGC CGG CAC TAG
Lys Leu Gly Pro Gln Pro Ala Asp ""
AAG CTG GGT CCG CAG CCG GCT GAC TAG

3

*** denotes a stop codon. Such expression vectors can be prepared by synthesising oligonucleotides corresponding to the desired extension and fitting these to the 3'-end of the LTB gene. The resulting extended LTB gene is provided in an expression vector with appropriate transcriptional and translational control elements.

The fusion protein that is expressed can be isolated. Where a fusion protein is expressed with a leader sequence, the protein will have been exported from the cytoplasm of the cell in which it is expressed. A fusion protein expressed with a natural LTB leader sequence, for example, can therefore be isolated and purified from the periplasm of E. coli.

The purified fusion protein, killed toxigenic strains of E. coli in which the fusion protein has been expressed and attenuated live vaccines capable of expressing the fusion protein can each be used as vaccines. The invention consequently further provides a vaccine in which an active principle is selected from:

(i) a fusion protein which comprises LTB having an antigen or epitope from a pathogen responsible for a human or animal disease fused to the carboxy-terminus of LTB;

(ii) a toxigenic strain of E. coli in which the said fustion protein has been expressed and which has been killed; and

(iii) an attenuated live vaccine capable of expressing the said fusion protein.

The vaccines typically also comprise a physiologically acceptable carrier or diluent. Conventional formulations, carriers and diluents may be employed. A suitable attenuated live vaccine may be an attenuated microorganism having a non-reverting mutation in each of two discrete genes in its aromatic biosynthetic pathway. Such microorganisms are described in EP-A-0322237. The microorganism is typically a pathogenic bacterium, for example from the genus Salmonella such as S. typhi, S. typhimurium, S. dublin or S. cholerasius.

The non-reverting mutations may occur in any of the aroA, aroC, aroD and aroE genes. Preferably one of the non-reverting mutations is in the aroA gene. A suitable attenuated microorganism may harbour an expression cassette encoding a fusion protein such that the fusion protein can be expressed by the microorganism. For reliable expression through generations of the microorganism, an expression cassette should be stably inherited in the absence of antibiotic selection.

A vaccine may be administered by any convenient route. The choice of whether an oral route or a parenteral route, such as subcutaneous, intravenous or intramuscular administration, is adopted, of the dose and of the frequency of vaccination depends upon the purpose of the vaccination, whether a human or animal is being treated and the condition of the human or animal to which the vaccine is to be given.

Typically, however, the fusion protein is administered in an amount of from 1-1000$\mu$g per dose, more preferably from 10-100$\mu$g per dose, by either the oral or the parenteral route. For attenuated S. typhi, on the other hand, a dosage of $10^9$ to $10^{11}$ S. typhi organisms per dose is generally convenient for a 70kg adult human patient, typically via the oral route.

The following Examples illustrate the invention. In the accompanying drawings:

Figure 1 shows the structure of pTRH5, pTRH6, pTRH13 and pTRH14 in which __ denotes the LTB gene, ⊏⊐ denotes the central core region of the STa2 gene and ▨ denotes the carboxy-terminal region of the STa2 gene;

Figure 2 shows the immunoreactivity of the hybrid proteins expressed by E. coli K-12 strain G6 harbouring pTRH5 or pTRH6, in which the y-axis denote log toxin titre, ■ denotes monoclonal antibody against STa2 and ▨ denotes monoclonal antibody against LTB; and

Figure 3 shows the results of an analysis for binding of anti-(hybrid protein) antibodies to ST determinants, in which the y-axis denotes absorbance at 450 nm; ■ denotes coating: hybrid antigen, probing antibody: pre-immunisation rabbit serum; ▨ denotes coating: LTB, probing antibody: serum from immunised rabbit, and ▦ denotes coating: hybrid antigen, probing antibody: immunised rabbit serum.

## EXAMPLE 1

The construction of recombinant LTB-ST hybrid proteins was achieved by fusing the gene encoding the B subunit of heat-labile enterotoxin (LT) to two different portions of the gene encoding heat-stable enterotoxin (ST). This entailed the introduction of linker sequences into the ST gene and the use of a modified LTB gene, both of which have been described previously (Sanchez et al, Gene 64, 265-275, 1988; Sanchez et al, FEBS Letters, 208, 194-198, 1986; Sandkvist et al, J. Bacteriol, 169, 4570-4576, 1987). The STa2 gene was isolated as an AvaII/BamH1 or DdeI/BamH1 fragment from pSLM4 (Moseley et al, Infect. Immun. 39, 1167-1174, 1983). The Ava II- or Dde I-cut ends were fitted with oligonucleotides A or B (Sanchez et al, 1986; Sanchez et al, 1988), thus producing an end complementary for EcoR1-cut DNA,

A:        AATTCGCCCGG          pJS6:  AvaII-EcoR1

          GCGGGCCCAG

          ――――― ―――――

               SmaI

B:        AATTCGCCCGGGTCC      pJS7:DdeI-EcoR1

       .  GCGGGCCCAGGAGT

          ――――― ―――――

               SmaI

and introducing a SmaI site within each extension. The fragments were then ligated into the vector pUC18 that had been cut with EcoR1 and Bam H1, thus producing pJS6 and pJS7.

Construction of pMM138 involved the isolation of the LTB gene from pWD615 (Dallas, Infect. Immun. 40, 647-652, 1983) as an EcoR1/HindIII fragment and its subsequent ligation into pMMB66EH (Furste et al, Gene 48, 119-31, 1986) to produce pMMB68. The SpeI site incorporating the stop codon of the hLTB gene was mutated to a HindIII site as described by Sandkvist et al, to yield plasmid pMMB138.

Plasmids pJS6 and pJS7 were digested with EcoR1 and SmaI. Plasmid pMMB138 was cut with HindIII, the large DNA fragment isolated and the projecting ends filled by T4 DNA polymerase (0.1 U/μl) and 100 nM dATP, dCTP, dGTP and dTTP. After precipitating the DNA fragment, it was further cut with EcoR1 and the smaller fragment, coding for hLTB was isolated. This was mixed with the EcoRI/SmaI digested pJS6 and pJS7 at a fragment:vector ratio of 10:1 (w/w), heated to 70°C for 2 minutes and then allowed to incubate at room temperature. After addition of ATP (1mM), BSA (29 μg/ml), DTT (4mM), and T4 DNA ligase (1.4 u/μl) the mixture was incubated at 4°C overnight to ligate the DNA (final concentration of DNA was 63 ng/μl).

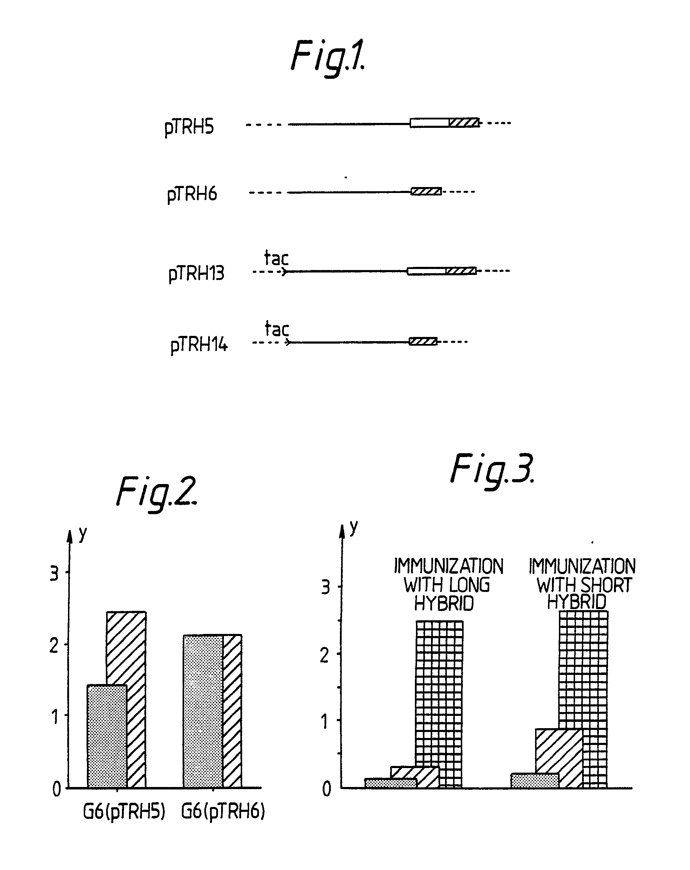

Competent E. coli K-12 strain G6 (Hardy et al, Proc. Natl. Acad. Sci. 85, 7109-7113 1988) were transformed with the ligation mixtures. Transformants producing material that bound to GM1-coated microtitre wells and that was recognised by monoclonal antibodies against hLTB and STa2 were grown up, the plasmid DNA isolated, and the restriction pattern checked against that predicted. Plasmid pTRH5 was derived from a fusion of the gene coding for hLTB and pJS7; pTRH6 arose from a similar fusion with pJS6 (Figure 1). The predicted joint sequences of the resulting hybrid proteins were:

                   +102
pMMB138            GluLysLeuAlaProGlnLysArgTrpSTOP

                   +102          +19
pTRH5              GluLysLeuGlyProGlnAspAla...

                   +102       +49
pTRH6              GluLysLeuGlyProGluSerMet...

The number 102 denotes the number of the hLTB amino acid. The ST amino acid residues are underlined and the number of the first is given. For the hybrid protein obtained from pTRH5, both the central core and the carboxy-terminal region of ST were present. Only the toxin region of ST was fused to hLTB in the recombinant protein expressed by plasmid pTRH6. E. coli CC118 harbouring pMMB138, pTRH5 or pTRH6 were deposited on 6 December 1989 at the National Collection of Industrial and Marine Bacteria, Aberdeen, GB under accession nos. NCIMB 40231, NCIMB 40232 and NCIMB 40233 respectively.

The fusion proteins were obtained from periplasmic fractions of the E. coli K-12 stain G6 harbouring plasmid pTRH5 or pTRH6. Periplasmic fractions were isolated by lysozyme-ethylene diamine tetraacetic acid (EDTA) treatment and then analysed by sodium dodecyl sulphate (SDS)-polyacrylamide gel electrophoresis (PAGE). The migration position of the recombinant proteins corresponded to the molecular weights of chimaeric proteins containing the different lengths of STa2 fused to LTB.

EXAMPLE 2

The hybrid genes constructed in Example 1 which encode the hLTB-ST fusion protein were introduced into a controlled expression vector pMMB66EH (Furste et al, 1986). This put transcription under the control of the tac promoter and allowed synthesis to be regulated and the proteins hyper-produced by addition of isopropyl-$\beta$-D-thiogalactopyranoside (IPTG). The resulting plasmids were pTRH13 and pTRH14 which produced the fusion proteins also produced by TRH5 and pTRH6 respectively (Figure 1).

In more detail, pTRH5 was digested with restriction endonucleases EcoR1 and HindIII and a 0.65kb fragment was isolated. Similarly, pTRH6 was cleaved with EcoR1 and HindIII and a fragment of 0.56kb was obtained. pMMB66 was treated with EcoRI and HindIII, mixed with the isolated fragment from pTRH5 or pTRH6 and the DNA ligated with T4 DNA ligase to create pTRH13 (insert from pTRH5) and pTRH14 (insert from pTRH6). Transformants carrying the new plasmids were selected on the basis of their ability to produce, in response to the addition of 1mM IPTG, fusion proteins that would bind to GM1-coated microtitre plates and were recognised by monoclonal antibodies against LTB and ST.

Expression of the hybrid proteins in E. coli K-12 strain G6 carrying pTRH13 and pTRH14 resulted in synthesis of two polypeptides with molecular weights corresponding to the expected intact hybrid proteins. Periplasmic samples from E. coli pTRH13 and pTRH14 were precipitated with ammonium sulphate (25-40% saturated), and dialysed against 1mM sodium phosphate pH7.2. The sedimentable aggregates generated during dialysis were collected by centrifugation and redissolved in 100mM sodium phosphate pH7.2, and then analysed by SDS-PAGE.

SDS-PAGE showed that the LTB-ST hybrid proteins retained an important property of LTB. They remained as discrete, stable pentamers when unheated prior to analysis by SDS-PAGE. However, they dissociated into monomers when boiled. This indicated that the attachment of ST to LTB did not interfer with a critical structural feature of LTB that lends it stability and resistance to proteolysis.

EXAMPLE 3: Recognition of LTB-STa2 hybrids by anti-LT and anti-ST antibodies

The immunoreactivity of the LTB-STa2 hybrids was evaluated in a GM1-based enzyme-linked immunosorbant assay (GM1-ELISA) using neutralising monoclonal antibodies against native LTB and ST to detect bound antigen. The assay is dependent on the ability of the hybrids to bind to GM1-ganglioside, and as such, a positive result provides unequivocal evidence that the hybrids have retained the receptor-binding property characteristic of LTB. The results are shown in Figure 2. In Figure 2 it can be seen that both the long and short hybrids were strongly detected by neutralising anti-LT and anti-ST antibodies. We therefore conclude that the hybrids exhibited several important structural features, including receptor GM1-binding and strong recognition by antitoxin antibodies, that are likely to play a vital role in their use as effective immunogens.

EXAMPLE 4: Toxicological properties

The purified LTB-ST hybrids were tested for ST toxicity in infant mouse assays. It was found that the shorter hybrid, encoded by pTRH14, was toxic. However, the longer and more stable construct, encoded by pTRH13 had very low toxicity indeed. No detectable toxicity was observed when 725 ng was injected intragastrically into infant mice, in which the sensitivity of the assay for native ST was 5 ng/mouse. Thi s indicated that the longer LTB-ST hybrid was attenuated to at least 0.7% of the native toxicity.

EXAMPLE 5: Immunogenicity of LTB-STa2 hybrids

Immunisation of rabbits with partialy purified preparations of the long and short hybrids gave high titre antibody responses to LTB and significant responses to ST. Rabbits were immunised with each hybrid protein employing a simple regimen of three injections, with Freunds Complete Adjuvant for priming and

6

Freunds Incomplete Adjuvant for boosters. Sera from rabbits immunized with either of the two hybrids elicited strong anti-LTB antibody responses with titres over 40000. That the hybrids elicited antibody responses to the ST moiety was demonstrated by two experimental approaches.

(1) An inhibition ELISA was used in which microtitre plates were coated with a chemically conjugated cholera toxin (CT) B subunit/ST hybrid. Polyclonal antisera directed against CTB was shown to have no effect on the binding of a mouse monoclonal antibody against ST. In contrast however, the serum obtained from rabbits immunized with the recombinant hybrids exhibited a strong inhibitory effect on the binding of the mouse monoclonal antibody. This suggests the presence of competing anti-ST antibodies in the rabbit immune system.

(2) A direct ELISA to discriminate between anti-LTB and anti-ST antibodies in the sera obtained from rabbits immunized with recombinant hybrids was developed. Microtitre plates were first coated with either recombinant hybrids or purified LTB and then all B subunit epitopes blocked by the addition of a high titre anti-LTB polyclonal mouse serum (RJ11). This was followed by the addition of sera from the rabbits. The binding of rabbit IgG was quantified with a horse radish peroxidase (HRO)-conjugated antibody. The results are shown in Figure 3.

In wells coated with LTB, the mouse serum efficiently blocked the subsequent binding of rabbit anti-LTB antibodies from the hybrid immune serum. In spite of this blocking effect antibodies in sera from rabbits immunized with the LTB-STa2 bound strongly to wells coated with the recombinant hybrids. This strongly suggests that the sera from immunized rabbits contains antibodies directed against the ST determinant.

The biological significance of these results has been investigated further, using the infant mouse assay of Example 4 to test whether these anti-ST responses can neutralize native ST toxin. Initial observations suggest that sera from rabbits immunized with the longer LTB-ST hybrid (pTRH13 gene product) are capable of neutralizing ST. The assay involved preincubation of native ST (40ng/ml) with serial dilutions of serum (e.g. 1/10, 1/60) followed by intragastric injection of infant mice. At both these dilutions the immune serum completely inhibited ST toxicity.

Previous findings by other researchers have suggested that ST-neutralizing antibodies are only elicited by ST-carrier conjugates which are ST-toxic. In view of our findings outlined above, we consider that our longer LTB-ST hybrid shows considerable promise as a LTB-ST toxoid antigen which should elicit protective antibodies against ST-producing ETEC.


EXAMPLE 6:

Plasmid pMMB138 (Sandkvist et al, 1987) encoding a mutant B subunit gene, etx B138, was used to insert a series of three oligonucleotide sequences which coded for the linker sequence, Lys Leu Gly Pro Gln, and convenient restriction sites for insertion of other oligonucleotides or DNA sequences specifying epitopes or antigens.

Plasmid pMMB138 was restricted with HindIII to give two DNA fragments whose recessed ends were filled in with the aid of T4 DNA polymerase. The larger of the two DNA fragments was then purified and ligated to three different annealed duplexes of synthetic oligonucleotides to generate three B subunit fusion vectors, designated pTRH100, pTRH101 and pTRH102. The sequences of oligonucleotides introduced were:

```
I     5' - GGGTCCGCAGGCCGGCGACTAGTGGATCCTCTAGA - 3'
      3' - CCCAGGCGTCCGGCCGCTGATCACCTAGGAGATCT - 5'


II    5' - GGGTCCGCAGGGCCGGCACTAGTGGATCCTCTAGA - 3'
      3' - CCCAGGCGTCCCGGCCGTGATCACCTAGGAGATCT - 5'


III   5' - GGGTCCGCAGCCGGCTGACTAGTGGATCCTCTAGA - 3'
      3' - CCCAGGCGTCGGCCGACTGATCACCTAGGAGATCT - 5'
```

Oligonucleotide duplexes I, II and III were used to generate pTRH100, pTRH101 and pTRH102

7

respectively. The fusion joints at the 3'-end of the gene encoding the B subunit of Escherichia coli heat-labile enterotoxin, the amino acids encoded by the oligonucleotide inserts and restriction endonuclease sites are shown below. Each vector contains a single NaeI site in one of three codon reading frames for insertion of additional DNA fragments or synthetic oligonucleotides. All cloning steps were carried out using standard procedures. Correct ligation and orientation of the fragments was analysed by fine restriction enzyme mapping.

+102

GluLysLeuGlyProGlnAlaGlyAsp***

pTRH100    GAAAAGCTGGGTCCGCAGGCCGGCGACTAGTGGATCCTCTAGAAGCTT

NaeI    SpeI    BamHI    XbaI    HindIII


+102

GluLysLeuGlyProGlnGlyArgHis***

pTRH101    GAAAAGCTGGGTCCGCAGGGCCGGCACTAGTGGATCCTCTAGAAGCTT

NaeI    SpeI    BamHI    XbaI    HindIII


+102

GluLysLeuGlyProGlnProAlaAsp***

pTRH102    GAAAAGCTGGGTCCGCAGCCGGCTGACTAGTGGATCCTCTAGAAGCTT

NaeI    SpeI    BamHI    XbaI    HindIII


Number +102 above the predicted amino acid sequence corresponds to the amino acid residue 102 in the mature B subunit (Leong et al, Infect. Immun. 48, 73-7, 1985).


EXAMPLE 7

A hybrid protein containing the B subunit fused to the (NANP)3 repetitive epitope of the malaria parasite Plasmodium falciparum was produced by inserting an oligonucleotide sequence coding for the (NANP)3 epitope into the NaeI site of plasmid pTRH102.

Plasmid pTRH102 was restricted with NaeI and BamHI and the larger fragment was purified. Two synthetic oligonucleotide sequences (shown below as A) encoding three tandem repeats of amino acids NANP were annealed together and digested with BamHI. The digested oligonucleotide duplex and pTRH102-NaeI/BamH1 fragment were ligated together and transformed into Escherichia coli K12 strain G6 (Enequist, et al., Eur. J. Biochem. 116, 227-33, 1981).

Recombinant plasmids were purified and their correct ligation and orientation were confirmed by fine restriction enzyme mapping. One of the correct plasmids was designated pTRH17.4. The nucleotide sequence and predicted amino acids encoded at the 3' end of the gene are shown below (B). Induction of the fusion product yielded a protein with the following properties:

(i) recognition of receptor GM1-ganglioside
(ii) assembly into defined oligomers
(iii) reaction by monoclonal antibodies against the LTB subunit and against the epitope (NANP)3 using Western-blotting and GM1-bound enzyme linked immunosorbant assays.
(iv) exported to the periplasm of Escherichia coli.

8

A.          5'-TAACGCTAACCCGAATGCGAACCCAAACGCAAACCCGTGAGGA
TCCGGGGGGGAA-3'
3'-ATTGCGATTGGGCTTACGCTTGGGTTTGCGTTTGGGCACT
CCTAGGCCCCCC-5'
BAMH1


+102
GluLysLeuGlyProGlnProAsnAlaAsnProAsnAlaAsnPro
AsnAlaAsnPro***

B. pTRH17.4   GAAAAGCTGGGTCCGCAGCCTAACGCTAACCCGAATGCGAACCCAAA
CGCAAACCCGTGAGGATCC


Number +102 corresponds to amino acid 102 in the mature sequence of the B subunit of Escherichia coli heat-labile enterotoxin. The underlined nucleotides in pTRH17.4 are derived from the synthetic oligonucleotide in A.


**Claims**

1. A DNA sequence encoding a fusion protein which comprises subunit B of E. coli heat-labile toxin (LTB) having an antigen or epitope from a pathogen responsible for a human or veterinary disease fused to the carboxy-terminus of LTB.

2. A DNA sequence according to claim 1, wherein a linker sequence is provided between the carboxy-terminus of LTB and the said antigen or epitope.

3. A DNA sequence according to claim 1, wherein the fusion joint comprises the amino acid sequence -Lys-Leu-Gly-Pro-Gln-.

4. A DNA sequence according to claim 1, wherein the LTB is fused to the core and toxin regions or to the toxin region of E. coli heat-stable toxin (ST).

5. A vector which comprises a DNA sequence as defined in claim 1 and which is capable, in a transformed host, of expressing the said fusion protein.

6. A vector according to claim 5, which is a plasmid.

7. A host transformed with a vector as claimed in claim 5.

8. A host according to claim 1, which is a transformed strain of E. coli.

9. A host according to claim 1, which is an attenuated live vaccine.

10. A fusion protein which comprises LTB having an antigen or epitope from a pathogen responsible for a human or veterinary disease fused to the carboxy-terminus of LTB.

11. A process for the preparation of a fusion protein comprising LTB having an antigen or epitope from a pathogen responsible for a human or veterinary disease fused to the carboxy-terminus of LTB, which process comprises culturing a transformed host as claimed in claim 7 under such conditions that the fusion protein is expressed.

12. A vaccine in which an active principle is selected from:

(i) a fusion protein which comprises LTB having an antigen or epitope from a pathogen responsible for a human or animal disease fused to the carboxy-terminus of LTB;

(ii) a toxigenic strain of E. coli in which the said fusion protein has been expressed and which has been killed; and

(iii) an attenuated live vaccine capable of expressing the said fusion protein.

13. An expression vector which encodes LTB, the LTB having a carboxy-terminal extension and the coding sequence for the extension having a restriction site therein.

14. A process for the preparation of a fusion protein comprising LTB having an antigen or epitope from a pathogen responsible for a human or veterinary disease fused to the carboxy-terminus of LTB, which process comprises

(i) inserting a DNA sequence encoding the said antigen or epitope into a vector as claimed in claim

9

13 at the said restriction site; and

(ii) culturing a host harbouring the resultant vector under such conditions that the fusion protein is expressed.

# Fig.1.

pTRH5

pTRH6

pTRH13  tac

pTRH14  tac

# Fig.2.

# Fig.3.

IMMUNIZATION WITH LONG HYBRID

IMMUNIZATION WITH SHORT HYBRID